# EUROPÄISCHE PATENTSCHRIFT

(11) **EP 1 595 605 B1**
(45) Veröffentlichungstag und Bekanntmachung des Hinweises auf die Patenterteilung: **05.05.2010**
(21) Anmeldenummer: 05008822.8
(22) Anmeldetag: 22.04.2005
(51) Int. Cl.: B05D 7/22, B05D 5/04, B05D 3/14

(54) **Verfahren zur Hydrophilisierung von Oberflächen fluidischer Bauteile und Systeme**
Method for coating with a hydrophilic composition fluid transport elements and apparatus comprising said fluid transport elements
Procédé de revêtement avec une composition hydrophile d'éléments de transports d'un fluide et appareil comprenant lesdits éléments de transport d'un fluide

(30) Priorität: 26.04.2004 EP 04009817
(43) Veröffentlichungstag der Anmeldung: 16.11.2005
(73) Patentinhaber: Roche Diagnostics GmbH, 68305 Mannheim (DE); F.HOFFMANN-LA ROCHE AG, 4070 Basel (CH)
(72) Erfinder: Weis, Léonie, Dr., 8045 Graz (AT); Werkl, Dietmar, 8010 Graz (AT); Leiner, Marco, Dr., 8045 Graz (AT); Ziegler, Werner, 8043 Graz (AT)

(56) Entgegenhaltungen:
- EP-A- 0 379 156
- US-A- 3 874 850
- US-A- 4 752 426
- US-A- 5 212 000
- US-B1- 6 447 835

## Beschreibung

### Gebiet der Erfindung:

Die Erfindung betrifft fluidische Systeme, welche aus zumindest einem sensorischen Element als fluidischem Bauteil und einem daran angrenzendem weiteren fluidischen Bauteil bestehen, auf deren inneren Oberflächen ein Film desselben hydrophilen Polymers vorliegt, und Verfahren zur Herstellung solcher fluidischer Systeme, insbesondere um deren Oberflächeneigenschaften, wie beispielsweise die Benetzbarkeit für wässrige Lösungen, zu modifizieren.
Weiterhin betrifft die Erfindung sensorisches Module zur Bestimmung physikalischer und/oder chemischer Parameter einer Flüssigkeit, welche mehrere sensorische Elemente sowie ein fluidisches Teilsystem zum Transport der Flüssigkeiten zu den sensorischen Elementen hin bzw. von diesen weg umfassen und in welchen ein solches fluidisches System enthalten ist.
Schließlich betrifft die Erfindung Analysesysteme zur Bestimmung mindestens eines physikalischen oder chemischen Parameters einer Flüssigkeit, insbesondere einer Körperflüssigkeit, welche mindestens ein sensorisches Element zur Bestimmung dieses Parameters und ein fluidisches System aus fluidischen Bauteilen besitzen und in welchen ein solches fluidisches System enthalten ist. Solche Analysesysteme können insbesondere zur Bestimmung diagnostisch relevanter Parameter aus Körperflüssigkeiten eingesetzt werden.

### Stand der Technik:

Moderne Analysesysteme zur Bestimmung physikalischer oder chemischer Parameter einer Flüssigkeit besitzen häufig komplexe fluidische Systeme, welche einen möglichst störungs- und verschleppungsfreien Transport der zu untersuchenden Flüssigkeit zu den sensorischen Elementen hin und nach der Bestimmung der Parameter einen Abtransport der Flüssigkeitsprobe von den sensorischen Elementen weg gewährleisten müssen.
Solche Analysesysteme werden insbesondere in der klinischen Diagnostik eingesetzt, wo sie insbesondere zur Blutgasanalyse oder zu sonstigen Messungen an in flüssiger Form vorliegenden Proben verwendet werden. Solche Systeme werden beispielsweise zur Bestimmung des Sauerstoff- oder Kohlendioxidpartialdrucks von Blut, der Hämoglobinparameter wie Gesamt-Hämoglobin, Oxyhämoglobin, Carboxyhämoglobin oder Methämoglobin von Vollblut oder hämolysiertem Blut, des Hämatokrit-Wertes von Vollblut sowie des pH-Werts oder der Konzentration von Ionen, beispielsweise Lithium, Natrium, Kalium, Magnesium, Calcium oder Chlorid oder speziellen Metaboliten wie Glukose, Harnstoff, Kreatinin oder Laktat in physiologischen Flüssigkeiten eingesetzt. Solche komplexen Analysesysteme besitzen zur Bestimmung der jeweiligen Parameter häufig verschiedene sensorische Elemente, welche für viele Bestimmungen eingesetzt werden. Solche sensorischen Elemente sind beispielsweise elektrochemische oder optische Sensoren zur Bestimmung der Gaswerte, des pH-Wertes, der Ionenwerte und der Metabolitwerte oder optische Messeinheiten zur Bestimmung der Hämoglobinwerte.
Das US-Patent US 3,874,850 (Sorensen et al.) beschreibt ein solches Analysesystem zur Blutanalyse. Dieses System weist mehrere sensorische Elemente auf, welche zur Bestimmung des pH-Wertes, der Partialdrücke von Sauerstoff und Kohlendioxid und des Hämoglobingehaltes einer Blutprobe dienen. Diese sensorischen Elemente sind durch ein komplexes fluidisches System aus verschiedensten fluidischen Bauteilen wie Schläuchen, Vorrats- und Abfallbehältern, Pumpen oder Ventilen verbunden, wobei bei diesem System die Probe mit Hilfe von Spritzen oder Kapillaren vom Patienten zum Analysesystem transportiert wird.
Neben derartigen Systemen sind auch Systeme bekannt, welche in unmittelbarer Nähe des Patienten verwendet werden können und bei welchen die Flüssigkeitsprobe mittels eines Schlauchsystems direkt vom Patienten in das Analysesystem überführt wird. Ein derartiges System wird beispielsweise in US 6,080,583 (von Bahr) beschrieben.
Viele Analysesysteme enthalten neben den fluidischen Teilsystemen zum Transport der Probenflüssigkeit und/oder von Qualitätskontrollmedien häufig auch weitere fluidische Teilsysteme, beispielsweise zum Transport flüssiger oder auch gasförmiger Kalibriermedien und/oder Wasch- oder Reinigungsmedien aus Vorratsbehältern hin zu den sensorischen Elementen und von dort weg zu Abfallbehältern. Dabei wird der Transport der verschiedenen Medien mittels Pumpen und Ventilen über teilweise unterschiedliche Wege gesteuert.
Ein weiteres Beispiel für Systeme zur Bestimmung mehrerer diagnostisch relevanter Parameter aus Probenflüssigkeiten mit einem komplexen fluidischen System ist das Analysesystem Omni S von Roche Diagnostics GmbH. Das modular aufgebaute Parameterprofil dieses Systems umfasst Blutgase, Elektrolyte, Gesamthämoglobin, CO-Oximetrie, Metabolite sowie Bilirubin und benötigt einen geringen Bedarf an Probenvolumen, so dass es sich besonders für den Einsatz auf der Neugeborenenstation eignet.

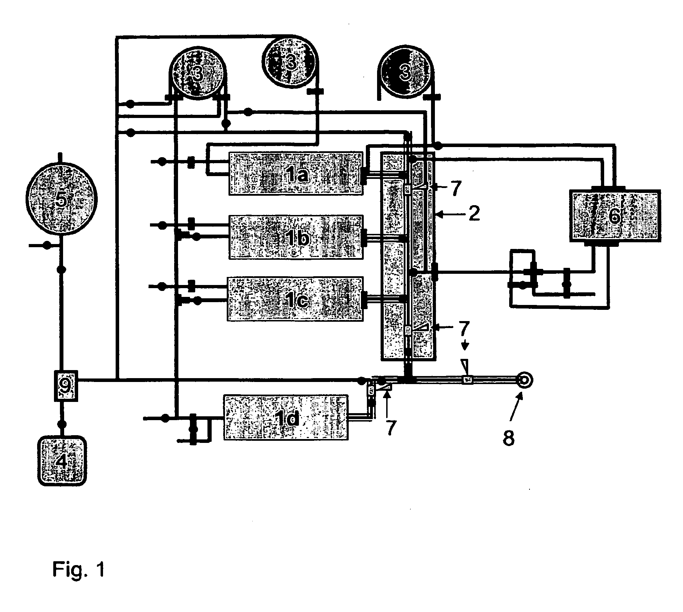

Figur 1 zeigt eine schematische Übersicht des fluidischen Systems des Omni S-Systems. Die Verbindungslinien zwischen den einzelnen fluidischen Bauteilen stellen hierbei fluidische Verbindungen dar, welche je nach Einsatzzweck als Schläuche oder Rohre ausgebildet sein können. Die innerhalb dieser fluidischen Verbindungen dargestellten kleinen Kreise stellen Ventile dar, mit deren Hilfe der Flüssigkeitstransport gesteuert werden kann. Diese sind meist als Quetschventile ausgebildet. Zu den fluidischen Verbindungen senkrecht stehende Balken stellen Ankopplungselemente dar, mit welchen fluidische Baugruppen miteinander in Kontakt gebracht werden können. Die als dreifache Linie gezeichneten fluidischen Verbindungen stellen den Probenweg einer Flüssigkeitsprobe vom Einfüllmund 8 bis zu den sensorischen Modulen 1a bis 1d dar. Die Bereiche 1a bis 1d symbolisieren jeweils sensorische Module, welche aus mehreren sensorischen Elementen bestehen können. So kann das sensorische Modul 1a sensorische Elemente zur Bestimmung von Metaboliten wie Laktat, Harnstoff oder Glukose, das sensorische Modul 1b ionenselektive Elektroden zur Bestimmung von Calcium, Kalium, Natrium oder Chlorid, das sensorische Modul 1c Blutgassensoren zur Bestimmung des Partialdrucks von Sauerstoff und Kohlendioxid sowie zur Bestimmung des pH-Wertes und das sensorische Modul 1d sensorische Elemente zur Bestimmung von Hämoglobin und Hämoglobinderivaten enthalten. Die zu untersuchende Probe wird über den Einfüllmund 8 in das fluidische System des Analysesystems eingebracht und dort entlang des Probenwegs zu den verschiedenen sensorischen Modulen 1a bis 1d transportiert, wobei die Probe während des Transports zusätzlich aliquotiert wird. Diese Funktion wird durch die im Probenweg befindlichen Ventile übernommen. Flüssigkeiten zu Qualitätskontrollzwecken können ebenfalls über den Einfüllmund eingebracht und auf dem gleichen Probenweg wie die Proben selbst zu den sensorischen Modulen transportiert werden. Der Verlauf des Flüssigkeitstransportes entlang des Probenwegs wird durch die physikalische Probensensoren 7 überwacht, welche unter anderem Auskunft über den Füllstand des Systems geben. Eine Aufteilung der eingebrachten Probe auf die sensorischen Module 1a bis 1c erfolgt innerhalb des Kanalsystems 2, in welches wiederum Ventile integriert sind. Flüssigkeiten zu Reinigungs- oder Kalibrierungszwecken liegen in den Flüssigkeitsbehältern 6 vor, welche über Schläuche und Rohre mit dem restlichen fluidischen System verbunden sind und über Ventile gesteuert in dieses eingespeist werden. Der Transport dieser Lösungen sowie auch der Probenlösungen erfolgt mittels der Peristaltikpumpen 3, welche über Ventile an das fluidische System angeschlossen sind. Verbrauchte Proben und Flüssigkeiten werden über das fluidische System von den sensorischen Modulen durch eine Vakuumpumpe 5 zu einem Sammelbehälter 9 transportiert, welcher in einen Abfallbehälter 4 mündet.

Solche modernen Geräte arbeiten oft mit sehr geringen Proben- und Flüssigkeitsmengen welche häufig im fluidischen System nochmals aliquotiert werden. Je nach Anzahl und Art der zu bestimmenden Parameter kann das benötigte Probenvolumen beispielsweise 50 bis 120 Mikroliter betragen. Es erfordert jedoch besondere Maßnahmen, um sicherzustellen, dass derartig kleine Probenvolumina ohne Kontaminationen zu den sensorischen Elementen des Analysesystems transportiert werden. Kontaminationen können beispielsweise durch im fluidischen System verbliebene Reste der vorherigen Proben beziehungsweise von Kontroll-, Kalibrations- oder Reinigungsmedien verursacht werden. Um solche Kontaminationen zu vermeiden, können zum Beispiel Wasch- und Trocknungsschritte zwischen die einzelnen Messwertsbestimmungen eingeschoben werden.
Bei sensorischen Elementen zur Bestimmung von Gasen, beispielsweise elektrochemischen oder optischen Gassensoren, aber auch bei optischen Meßsystemen, beispielsweise zur Hämoglobinbestimmung, besteht weiterhin die Gefahr, dass es zu Fehlmessungen aufgrund des Einschlusses von Gasblasen im fluidischen System, insbesondere im Bereich der sensorischen Elemente, kommt. So können bei der Bestimmung von gasförmigen Analyten in Flüssigkeiten mittels elektrochemischer Gas-Sensoren Probleme bei der Probenmessung bzw. bei der Kalibrierung oder Qualitätskontrolle auftreten, wenn die Probe bzw. das Qualitätskontroll- oder Kalibriermittel den flüssigkeitsführenden Bereich des sensorischen Elements nur unvollständig füllt oder wenn sich dort Gasblasen, beispielsweise Luftblasen, befinden. Gasblasen bilden sich insbesondere bei Vorliegen von uneinheitlichen inneren Oberflächen innerhalb des fluidischen Systems, welche unterschiedliche Benetzungseigenschaften durch Flüssigkeiten aufweisen. Besonders häufig kommt es an den Stellen des fluidischen Systems zur Bildung oder Festsetzung von Gasblasen, an welchen ein sprunghafter Übergang in den Benetzungseigenschaften der inneren Oberflächen der verschiedenen fluidischen Bauteile vorhanden ist. Dies ist beispielsweise dann der Fall, wenn Oberflächen aus unterschiedlichen Materialien aufeinander stoßen. Die fluidischen Systeme vieler Analysesysteme bestehen jedoch aus vielen einzelnen fluidischen Bauteilen aus unterschiedlichen Materialien, welche aneinander stoßende Oberflächen mit unterschiedlichen Benetzungseigenschaften besitzen. Weiterhin bestehen viele der fluidischen Bauteile solcher Analysesysteme aus Kunststoffen, welche sich durch eine geringe Hydrophilie oder eine Hydrophobie auszeichnen. Solche Kunststoffoberflächen lassen sich schlecht mit wässrigen Flüssigkeiten benetzen und neigen besonders zur Bildung oder Festsetzung von Gasblasen.
WO 02/070590 (Faure et al.) beschreibt Verfahren, die Oberflächen hydrophober Substrate zu hydrophilisieren, in dem ein bereits existierender Film eines hydrophilen Polymers auf ein Substrat aufgebracht wird. Bedingt durch die schlechte Handhabbarkeit solcher vorgefertigter sehr dünner Filme hydrophiler Polymere ist eine in WO 02/070590 offenbarte Aufbringung eines solchen vorgefertigten und mechanisch kaum belastbaren Films auf die inneren und somit nur sehr schwer zugänglichen Oberflächen fluidischer Bauteile wie beispielsweise Schläuche fertigungstechnisch sehr schwierig und aufwändig und daher ökonomisch nicht sinnvoll.
US 6,432,510 (Kim et al.) beschreibt Verfahren zur Verbesserung der Benetzungseigenschaften von Oberflächen, bei welchen zunächst die Oberfläche des Grundsubstrats mittels physikalisch-chemischer Verfahren aufgeraut wird. Auf diese angerauten Oberflächen wird anschließend mittels physikalischer Verfahren, beispielsweise durch Abscheidung der Beschichtungssubstanz aus der Gasphase, eine dünne Schicht einer Beschichtungssubstanz aufgetragen, welche eine gute Benetzbarkeit aufweist. Bei den Verfahren nach US 6,432,510 muss als kritischer Punkt die zuvor erzeugte Aufrauung des Grundmaterials auch nach dem Aufbringen der Beschichtungssubstanz erhalten bleiben, um eine Verbesserung der Benetzungseigenschaften zu erreichen. Fluidische Bauteile mit solch aufgerauten Oberflächen eignen sich jedoch nicht zum Einsatz in diagnostischen Analysesystemen, da der Kontakt physiologischer Flüssigkeiten mit derartig aufgerauten Oberflächen zu einer deutlichen Erhöhung des Kontaminationsrisikos und damit zu einem deutlich erhöhten Aufwand für zusätzliche Wasch- und Reinigungsschritte führt. Insbesondere ist an solchen Oberflächen das Risiko der Anlagerung zellulärer und makromolekularer Bestandteile der zu untersuchenden Flüssigkeit, insbesondere im Falle von Blut, stark erhöht.
US 5,212,000 (Rose et al.) beschreibt Beschichtungsverfahren zur Herstellung von Polymerschichten auf den Innenseiten von dünnen fluidischen Elementen durch Einbringen spezieller Polymerlösungen mit thixotropen Eigenschaften. Aufgrund dieser speziellen thixotropen Eigenschaften bleibt zumindest ein Teil der Polymerlösung auf der Innenseite der Membran als Beschichtung zurück. Allerdings ist die Verwendung solcher Verfahren nur auf speziellen Anwendungsgebieten und mit speziellen thixotropen Beschichtungslösungen durchführbar. Aufgrund dieser thixotropen Eigenschaften besteht insbesondere in Systemen, in welchen Flüssigkeiten mit teilweise sehr hohen Drücken durch die Lumina der fluidischen Elemente geleitet werden, die Gefahr, dass sich durch diese Druckeinwirkungen und/oder Volumenströme die Beschichtungssubstanz wieder verflüssigt und von der Oberfläche löst.
EP 0379156 (Fan) beschreibt Beschichtungsverfahren, bei welchen zunächst auf die Oberfläche einer medizinischen Vorrichtung, insbesondere eines Katheders eine Polyisocyanatlösung aufgebracht wird, diese (optional) eingetrocknet wird und anschließend eine weitere Lösung eines carboxylsäuregruppenhaltigen Polymers aufgebracht wird. Bei solchen zwei- oder mehrstufigen Verfahren mit mehreren beteiligten Reagenzienlösungen und chemischen Reaktionsschritten sind meist viele Prozessschritte notwendig, um zu gewünschten Beschichtung zu gelangen. Für eine möglichst einfache und kostengünstige Beschichtungstechnik ist es allerdings vorteilhaft, mit möglichst wenigen Verfahrensschritten und beteiligten Reaktionslösungen auszukommen.

US 4,752,426 (Cho) beschreibt Verfahren zur Hydrophilisierung von Oberflächen, bei welchen zunächst mittels einer Niedertemperatur-Plasmabehandlung chemisch reaktive Gruppen bzw. Radikale auf der Oberfläche erzeugt werden. Anschließend wird eine Monomer-Lösung auf diese Oberfläche aufgebracht. Die Monomere reagieren dort chemisch mit den chemisch reaktiven Gruppen bzw. Radikalen auf der Oberfläche, so dass letztlich auf der Oberfläche mittels Pfropfpolymerisation eine Beschichtung gebildet wird. Nachteilig bei diesen Verfahren ist, dass die Verfahrensschritte undbedingungen möglichst exakt aufeinander abgestimmt werden müssen. Beispielsweise müssen die Plasmabehandlungsparameter derartig gewählt werden, dass möglichst nur solche chemisch reaktive Gruppen bzw. Radikale auf der Oberfläche gebildet werden, welche auch aus Polymerisationskeime für die nachfolgende Pfropfpolymerisation dienen können.

### Aufgabe:

Aufgabe der vorliegenden Erfindung ist es daher, Verfahren zur Verbesserung der Oberflächenbenetzbarkeit der inneren Oberflächen von fluidischen Systemen bereitzustellen, welche die Nachteile des Standes der Technik vermeiden.

Aufgabe der vorliegenden Erfindung ist es insbesondere, möglichst einfache und kostengünstig durchzuführende Verfahren bereitzustellen, welche eine über eine lange Einsatzdauer stabile und gegenüber physikalischen oder chemischen Belastungen widerstandsfähige Hydrophilisierung der inneren Oberflächen fluidischer Systeme ermöglichen.

Aufgabe der vorliegenden Erfindung ist es weiterhin, fluidische Systeme bereitzustellen, bei welchen das Risiko einer Festsetzung oder Bildung von Gasblasen an den inneren Oberflächen dieser fluidischen Bauteile über eine lange Einsatzdauer hinweg reduziert werden kann.

Aufgabe der vorliegenden Erfindung ist es weiterhin, sensorische Module und Analysesysteme zur Bestimmung mindestens eines physikalischen oder chemischen Parameters einer Flüssigkeit bereitzustellen, bei welchen das Risiko einer Kontamination der Probenflüssigkeit, insbesondere durch die Festsetzung oder Bildung von Gasblasen oder sonstigen Substanzen an den inneren Oberflächen der fluidischen Systeme, über eine lange Einsatzdauer hinweg reduziert werden kann.

### Erfindungsgemäße Lösung:

Die Lösung dieser Aufgaben erfolgt erfindungsgemäß durch die Bereitstellung fluidischer Systeme aus zumindest einem sensorischen Element als fluidischem Bauteil und zumindest einem daran angrenzendem weiteren fluidischen Bauteil, wobei sowohl auf den inneren Oberflächen der flüssigkeitsführenden Bereiche des sensorischen Elements als auch des daran angrenzenden fluidischen Bauteils ein Film desselben hydrophilen Polymers vorliegt.

Die Lösung dieser Aufgaben erfolgt erfindungsgemäß durch die Bereitstellung sensorischer Module zur Bestimmung physikalischer und/oder chemischer Parameter einer Flüssigkeit, welche mehrere sensorische Elemente sowie ein fluidisches Teilsystem zum Transport der Flüssigkeiten zu den sensorischen Elementen hin bzw. von diesen weg umfassen und in welchen zumindest ein fluidisches System gemäß obigem Absatz enthalten ist.

Die Lösung dieser Aufgaben erfolgt erfindungsgemäß durch die Bereitstellung von Analysesystemen zur Bestimmung mindestens eines physikalischen oder chemischen Parameters einer Flüssigkeit, welche mindestens ein sensorisches Element zur Bestimmung mindestens eines physikalischen oder chemischen Parameters der Flüssigkeit und ein fluidisches System zum Transport von Flüssigkeiten zum sensorischen Element hin und/oder vom sensorischen Element weg umfassen und in welchen zumindest ein fluidisches System gemäß obigem Absatz enthalten ist.

Schließlich erfolgt die Lösung dieser Aufgaben durch die Bereitstellung von Verfahren zur Herstellung eines fluidischen Systems gemäß obigem Absatz, welche aus zumindest einem sensorischen Element als fluidischem Bauteil und zumindest einem daran angrenzendem weiteren fluidischen Bauteil bestehen und bei welchen sowohl auf den inneren Oberflächen der flüssigkeitsführenden Bereiche des sensorischen Elements als auch des daran angrenzenden fluidischen Bauteils ein Film desselben hydrophilen Polymers vorliegt, wobei die Herstellung eines Films desselben hydrophilen Polymers dadurch erfolgt, dass zunächst die inneren Oberflächen der fluidischen Bauteile einer physikalisch-chemischen Vorbehandlung unterzogen werden, anschließend die inneren Oberflächen der fluidischen Bauteile mit einer Lösung desselben hydrophilen Polymers in Kontakt gebracht werden,
anschließend die Lösung des hydrophilen Polymers durch ein gasförmiges Medium derartig ersetzt wird, dass zunächst die inneren Oberflächen der fluidischen Bauteile mit einem Teil der Lösung des hydrophilen Polymers benetzt bleiben und schließlich ein Film des hydrophilen Polymers durch Entzug des Lösungsmittels auf den inneren Oberflächen der fluidischen Bauteile verbleibt und die Herstellung des Filmes des hydrophilen Polymers zunächst auf den inneren Oberflächen einzelner fluidischer Bauteile oder kleinerer Baugruppen solcher fluidischer Bauteile erfolgt, welche anschließend zu dem fluidischen System gemäß obigem Absatz zusammengefügt werden.

Alternativ erfolgt die Lösung dieser Aufgaben durch die Bereitstellung von Verfahren zur Herstellung eines fluidisches Systems gemäß obigem Absatz, welche aus zumindest einem sensorischen Element als fluidisches Bauteil und zumindest einem daran angrenzendem weiteren fluidischen Bauteil bestehen und bei welchen sowohl auf den inneren Oberflächen der flüssigkeitsführenden Bereiche des sensorischen Elements als auch des daran angrenzenden fluidischen Bauteils ein Film desselben hydrophilen Polymers vorliegt, wobei die Herstellung eines Films desselben hydrophilen Polymers dadurch erfolgt, dass zunächst die inneren Oberflächen der einzelnen fluidischen Bauteile einer physikalisch-chemischen Vorbehandlung unterzogen werden, anschließend die inneren Oberflächen der fluidischen Bauteile mit einer Lösung desselben hydrophilen Polymers in Kontakt gebracht werden, anschließend die Lösung des hydrophilen Polymers durch ein gasförmiges Medium derartig ersetzt wird, dass zunächst die inneren Oberflächen der fluidischen Bauteile mit einem Teil der Lösung des hydrophilen Polymers benetzt bleiben und schließlich ein Film des hydrophilen Polymers durch Entzug des Lösungsmittels auf den inneren Oberflächen der fluidischen Bauteile verbleibt, wobei die physikalisch-chemische Vorbehandlung zunächst auf den inneren Oberflächen einzelner fluidischer Bauteile oder kleinerer Baugruppen solcher fluidischer Bauteile erfolgt, diese fluidischen Bauteile oder Baugruppen anschließend zu dem fluidischen System zusammengefügt werden und die nachfolgenden Verfahrensschritte an diesem zusammengefügtem fluidischen System erfolgen.

Zur Verdeutlichung der Erfindung werden im Folgenden einige Begriffe erläutert:

Als fluidisches Bauteil wird im Sinne der vorliegenden Anmeldung prinzipiell jedes Bauteil angesehen, welches dazu geeignet ist, Flüssigkeiten aufzunehmen, weiterzuleiten oder abzugeben. Insbesondere werden im Sinne der vorliegenden Anmeldung Bauteile als fluidische Bauteile angesehen, welche durch ihre spezielle räumliche Struktur dazu ausgelegt sind, Flüssigkeiten gezielt und definiert aufzunehmen, weiterzuleiten oder abzugeben. Fluidische Bauteile werden häufig als Komponenten eines fluidischen Systems eingesetzt und dienen hierbei dem Transport von Flüssigkeiten innerhalb dieses Systems. Solche fluidischen Bauteile können hierbei verschiedene Funktionen erfüllen, insbesondere können sie als Flüssigkeitsbehälter wie beispielsweise Flaschen, Reagenzienpacks, Abscheider oder Abfallbehälter, zum Flüssigkeitstransport wie beispielsweise Schläuche, Rohre, Kanäle oder flüssigkeitsführende Bereiche sensorischer Elemente, als Ventile oder Steuerelemente wie beispielsweise Quetschschlauchventile, Mischventile, Druckventile oder Ventil-T-Stücke, als Flüssigkeitsaufnahmevorrichtungen wie beispielsweise Nadeln, Rohre oder speziell geformte Einfüllmünder, zur Ankopplung verschiedener Bauteile wie beispielsweise Nippel, Dockelastomere, T- oder Y-Stücke, Kreuzungsstücke, Winkelstücke oder Steckverbindungen oder zu Dichtfunktionen wie beispielsweise Dichtelemente eingesetzt werden. Zur Erfüllung ihrer Funktion bestehen beispielsweise Schläuche aus elastischen Materialien und können insbesondere zum definierten Transport von Flüssigkeiten, beispielsweise durch Regulierung mittels Quetschventile, verwendet werden. Insbesondere können sie in Analysesystemen zum gezielten Transport der Probenflüssigkeiten zu den sensorischen Elementen und als einfach zu montierende Komponenten zum Abtransport der Flüssigkeiten, beispielsweise in einem Abfallbehälter, genutzt werden. Im Gegensatz hierzu werden starre Rohre oft aus Materialien mit bestimmten physikalischen Eigenschaften wie Transparenz, Gasdichtheit, Wärmeleitfähigkeit oder Reinigbarkeit hergestellt und als fluidische Bauteile häufig zum Flüssigkeitstransport für die Bereiche verwendet, welche nicht durch Quetschventilen reguliert werden müssen. Ankopplungen wie Stecknippel, T- oder Y-Stücke, Kreuzungsstücke, Winkelstücke oder Steckverbindungen werden als fluidische Bauteile häufig eingesetzt, um Übergänge zwischen anderen fluidischen Bauteilen wie Schläuchen, Rohren oder auch den flüssigkeitsführenden Bereichen sensorischer Elemente zu ermöglichen. Dockelastomere sind spezielle Ausführungen von Koppelsystemen, welche ein benutzerfreundliches häufiges An- und Abstecken einzelner Komponenten eines fluidischen Systems ermöglichen. Solche Koppelsysteme können zum Beispiel bei Reagenzienpacks verwendet werden, um mit einem Handgriff deren Ankopplung an das fluidische System zu ermöglichen. Solche Koppelsysteme können dazu verwendet werden, einzelne Komponenten des fluidischen Systems leicht zu entfernen und zu ersetzen. Eine spezielle Form von Flüssigkeitsaufnahmevorrichtungen sind so genannte Einfüllmünder. Diese dienen der Aufnahme externer Flüssigkeitsbehälter wie beispielsweise Kapillaren, Spritzen oder Adaptern. Über einen solchen Einfüllmund kann eine dichte fluidische Verbindung zwischen einem externen Probengefäß und einem diagnostischen Analysesystem hergestellt werden, so dass auf diese Weise Probenflüssigkeit sicher in das Analysesystem gespritzt oder gesaugt werden kann. Flüssigkeitsbehälter wie beispielsweise Flaschen oder Reagenzienpacks dienen der Aufbewahrung und Bereitstellung von Flüssigkeiten, beispielsweise von Kalibrierflüssigkeiten oder Reinigungs- und Konditionierlösungen. Aus diesen Flüssigkeitsbehältern können die Lösungen beispielsweise durch Ansaugen entnommen werden. Dichtungselemente werden insbesondere zur fluidischen Kopplung bei Übergängen von zwei nicht elastischen fluidischen Bauteilen eingesetzt. Fluidische Bauteile können auch komplexere Vorrichtungen zum Transport von Flüssigkeiten aufweisen, beispielsweise Kanalsysteme. Als fluidische Bauteile gelten im Sinne der vorliegenden Anmeldung auch die flüssigkeitsführenden Bereiche sensorischer Elemente. Hierunter versteht man die Bereiche sensorischer Elemente, welche mit der zu untersuchenden Flüssigkeit in direktem Kontakt stehen können und somit innere Oberflächen im Sinne der vorliegenden Anmeldung besitzen. Dies kann beispielsweise der flüssigkeitszu- und abführende Probenkanal innerhalb des sensorischen Elements sein oder auch die Teile des Sensors selbst, welche direkt mit der Flüssigkeit in Kontakt treten, beispielsweise die gasdurchlässigen Membranen elektrochemischer Gassensoren. Im Falle optischer Messverfahren, beispielsweise bei der Hämoglobinbestimmung, wird insbesondere auch der Teil des sensorischen Systems, welcher das zu analysierende Flüssigkeitsvolumen zum Zeitpunkt der Messwertsbestimmung enthält, als fluidisches Bauteil angesehen. Fluidische Bauteile werden häufig aus Materialien hergestellt, welche an die speziellen Anforderungen des fluidischen Bauteils angepasst sind. So werden beispielsweise Schläuche häufig aus Silikonkautschuken oder weichen Polyvinylchloriden, Rohre aus Polyvinylchloriden, Polyamiden oder Acrylnitril-Butadien-Copolymeren, Kanalsysteme aus Polycarbonaten, Polymethylmethacrylaten, Acrylnitril-Butadien-Copolymeren oder Styrol-Methmethacryl-Butadien-Copolymeren, Ankopplungen aus Polypropylenen, Polycarbonaten oder Polyamiden, Dockelastomere aus Nitrilkautschuken, Silikonkautschuken oder Fluorkautschuken, Einfüllmünder aus Silikonkautschuken oder Polyetheretherketonen, Flaschen und Flüssigkeitsbehälter aus Polyethylenen und Dichtungselemente aus Naturkautschuken, Silikonkautschuken oder Fluorkautschuken hergestellt.

Als fluidisches System wird im Sinne der vorliegenden Anmeldung eine Kombination mehrerer fluidischer Bauteile angesehen, welche in einer Weise miteinander verbunden sind, dass ein Flüssigkeitstransport zwischen diesen fluidischen Bauteilen möglich ist. Insbesondere sind solche fluidischen Systeme dazu geeignet, Flüssigkeiten aufzunehmen, weiterzuleiten oder abzugeben. Durch die Kombination verschiedener fluidischer Bauteile zu einem fluidischen System können die Funktionen einzelner fluidischer Bauteile miteinander kombiniert werden, so dass das entstehende fluidische System auch komplexe fluidische Funktionen ermöglichen kann. So kann beispielsweise durch eine geeignete Kombination von Schläuchen, Ventilen und Pumpen ein fluidisches System erzeugt werden, welches definierte Flüssigkeitsvolumina über eine definierte Strecke transportieren kann. Solche fluidischen Systeme können beliebig viele fluidische Bauteile enthalten und beliebig komplex sein. Als ein fluidisches System kann auch ein einziges Bauteil angesehen werden, welches durch seinen räumlichen Aufbau die Funktionen mehrerer fluidischer Bauteile übernehmen kann. Solche komplexen Bauteile können beispielsweise im Spritzgussverfahren hergestellt werden, wodurch insbesondere komplexe Kanalsysteme in einem einzigen Bauteil hergestellt werden können. Fluidische Systeme werden insbesondere in Analysesystemen eingesetzt, wobei auch die flüssigkeitsführenden Bereiche sensorischer Elemente als Bestandteil des fluidischen Systems angesehen werden.

Als Analysesystem wird im Sinne der vorliegenden Anmeldung jedes System angesehen, welches mindestens ein sensorisches Element zur Bestimmung mindestens eines physikalischen oder chemischen Parameters einer Flüssigkeit und ein fluidisches System zum Transport von Flüssigkeiten zum sensorischen Element hin und/oder vom sensorischen Element weg aufweist. Analysesysteme können sowohl mehrere voneinander unabhängige oder miteinander verbundene fluidische Teilsysteme sowie mehrere sensorische Einheiten oder Module aufweisen. Beispielsweise kann ein Analysesystem ein fluidisches Teilsystem für den Transport von Proben- oder Qualitätskontrollflüssigkeiten und ein fluidisches Teilsystem zum Transport flüssiger oder auch gasförmiger Kalibriermedien und/oder Wasch- oder Reinigungsmedien aufweisen, welche beispielsweise durch Ventile zu einem fluidischen Gesamtsystem verbunden werden können.

Als sensorische Elemente werden im Sinne der vorliegenden Anmeldung alle Vorrichtungen angesehen, mit welchen physikalische oder chemische Parameter einer Flüssigkeit bestimmt werden können. Dies können insbesondere Sensoren sein, welche direkt mit der zu untersuchenden Probe in Kontakt treten oder auch sensorische Systeme, welche die physikalischen oder chemischen Parameter indirekt, beispielsweise mittels optischer Transmissions- oder Streulichtmessungen, bestimmen. Sensoren der ersten Art können beispielsweise elektrochemische oder optische Sensoren zur Bestimmung der Gaswerte, des pH-Wertes, der Ionenwerte und der Metabolitwerte von Blutproben sein. Sensorische Systeme der zweiten Art können beispielsweise optische Messeinheiten zur Bestimmung der Hämoglobinwerte von Blutproben sein. Sensoren der ersten Art treten direkt mit der zu untersuchenden Flüssigkeit in Kontakt, so dass der flüssigkeitsführende Bereich solcher sensorischen Elemente die Funktion eines fluidischen Bauteils ausübt. Bei sensorischen Systemen der zweiten Art kann der Bereich des Systems, welcher dem Flüssigkeitstransport dient, insbesondere der Bereich des Systems, welcher das zu analysierende Flüssigkeitsvolumen zum Zeitpunkt der Messwertsbestimmung enthält, funktionell als fluidischer Bauteil angesehen werden. Beispielsweise können dies bei optischen Messsystemen spektroskopische Messstrecken innerhalb des fluidischen Systems oder Küvetten sein.

Als sensorische Module werden im Sinne der vorliegenden Anmeldung allgemein Kombinationen mehrerer sensorischer Elemente angesehen. Solche sensorischen Module können insbesondere mehrere sensorische Elemente innerhalb eines gemeinsamen Gehäuses aufweisen, welches ein gemeinsames fluidisches Teilsystem besitzt. Solche sensorischen Module können insbesondere in Form von Kassetten oder sensorischen Arrays ausgebildet sein, mit welchen eine Mehrzahl von physikalischen oder chemischen Parametern weitgehend gleichzeitig anhand einer einzigen Probe ermittelt werden kann. Solche sensorischen Module können beispielsweise ein fluidisches System mit einem einzigen fluidischen Eingang, einem durchgängigen oder auch verzweigten fluidischen Transportraum aus einem oder mehreren Kanälen und einen einzigen fluidischen Ausgang besitzen. Innerhalb dieses sensorischen Moduls können sich mehrere sensorische Elemente in Kontakt mit dem gemeinsamen fluidischen Transportraum befinden, welcher somit die Funktion eines gemeinsamen Probenkanals erfüllt.

Als innere Oberflächen eines fluidischen Bauteils oder eines fluidischen Systems werden im Sinne der vorliegenden Anmeldung die Oberflächen angesehen, welche in der räumlichen Form, in welcher das fluidische Bauteil zum Flüssigkeitstransport eingesetzt wird, mit der zu transportierenden Flüssigkeit in Kontakt kommen können. Dies sind beispielsweise die Innenwände von Schläuchen, Rohren oder Kanälen, welche von einer durchfließenden Flüssigkeit kontaktiert werden können. Dabei muss jedoch nicht notwendigerweise die gesamte innere Oberfläche des fluidischen Bauteils mit der Flüssigkeit in Kontakt kommen.

Als hydrophile Polymere im Sinne der vorliegenden Anmeldung werden polymere Substanzen angesehen, welche aus Monomerbausteinen des gleichen Typs oder unterschiedlicher Typen aufgebaut sind und hydrophile Eigenschaften aufweisen. Die Polymerketten solcher Polymere sind hydrophil oder weisen zumindest hydrophile Kettensequenzen auf. Solche hydrophilen Polymere besitzen chemische Gruppen mit hoher Affinität zu Wasser wie beispielsweise Hydroxy- oder Ether-Gruppen. Beispiele für hydrophile Polymere sind bestimmte Polyether wie bestimmte Polyethylenglycole oder bestimmte Polypropylenglycole, bestimmte Polysaccharide wie bestimmte Dextrane oder bestimmte Polyalkohole wie bestimmte Polyvinylalkohole. Insbesondere können auch bestimmte Polyether-Polyurethan-Copolymere als hydrophile Polymere eingesetzt werden. Ein großer Vorteil der vorliegenden Erfindung ist es, dass das hydrophile Polymer nicht erst auf der zu beschichtenden Oberfläche hergestellt werden muss, sondern die hydrophilen Polymerketten in Form einer Lösung auf die Oberfläche aufgebracht werden können. Hiermit unterscheidet sich die vorliegende Erfindung insbesondere von hydrophilen Beschichtungen, welche durch Polymerisation von Vorstufen, insbesondere durch Plasmapolymerisation oder Pfropfpolymerisation, auf einer Oberfläche hergestellt werden, da durch das erfindungsgemäße Verfahren, welches mit bereits auspolymerisierten hydrophilen Polymeren, welche im wesentlichen aus nicht kovalent quervernetzten Polymerketten bestehen, arbeitet, auf aufwändige Polymerisationsschritte verzichtet werden kann. Nachteilig an Beschichtungsverfahren mittels Polymerisation aus Vorstufen auf einer Oberfläche ist weiterhin, dass die Reproduzierbarkeit bei der Herstellung dünner Schichten von vielen Faktoren abhängt und damit nicht immer gewährleistet ist. Durch den Einsatz erfindungsgemäßer hydrophiler Polymere ist es möglich, ohne zusätzliche chemische Reaktionsschritte direkt und einfach eine hydrophile Beschichtung herzustellen. Hierbei müssen die Polymerketten nicht notwendigerweise untereinander quervernetzt vorliegen, um eine dauerhafte hydrophile Beschichtung zu erreichen. Durch die Verwendung erfindungsgemäßer Polymere können sich diese auch ohne kovalente Quervernetzungen untereinander zu einem haltbaren Film eines hydrophilen Polymers anordnen.

Besonders bevorzugt können daher hydrophile Polymere eingesetzt werden, welche aus nicht kovalent quervernetzten Polymerketten bestehen. Dies ist insbesondere deshalb vorteilhaft, da die Verwendung bereits auspolymerisierter hydrophiler Polymere mit weniger Aufwand verbunden ist als die Polymerisation von Vorstufen an der Oberfläche eines Substrates zu einem dünnen Film.

Als hydrophile Polymere im Sinne der Anmeldung gelten insbesondere bereits fertig auspolymerisierte Polymermoleküle, insbesondere längerkettige Polymermoleküle, welche nicht kovalent untereinander quervernetzt sind und somit in geeigneten Lösungsmitteln noch in ausreichender Menge gelöst werden können. Nicht kovalent quervernetzt ist hierbei so zu verstehen, dass die einzelnen Polymerketten im Wesentlichen nicht untereinander kovalent verknüpft sind. Es ist jedoch prinzipiell möglich, das nach Auftrag der erfindungsgemäßen nicht kovalent quervernetzten Polymerketten auf die zu beschichtende Oberfläche und der Bildung des Polymersfilms oder durch nachgeschaltete Vorgänge, z.B. während der Lagerung, nachträglich unspezifische kovalente Bindungen in geringem Maße zwischen einzelnen Polymerketten und/oder einzelnen Polymerketten und der Oberfläche entstehen können. Solche eventuell unspezifisch auftretenden Bindungen, wie sie beispielsweise in Folge einer Plasmabehandlung entstehen können, zwischen den einzelnen Polymerketten oder Polymerketten und Oberfläche sind jedoch nicht wesentlich für die Herstellung und/oder die erfindungsgemäßen Eigenschaften des Polymerfilms. Sie sind somit klar von den chemischen Bindungen zwischen einzelnen Molekülen und/oder Oberfläche im Falle erst auf den zu beschichtenden Oberfläche aus Vorstufen hergestellter Polymerfilme zu unterscheiden, bei welchen diese entstehenden chemischen Bindungen essentiell und entscheidend für die Herstellung und die Eigenschaften des gebildeten Polymerfilms sind.

Als Film im Sinne der vorliegenden Anmeldung wird eine weitgehend zusammenhängende und einheitliche Schicht einer Substanz auf einem Untergrund angesehen, welche insbesondere dadurch hergestellt wird, dass die aufzutragende Substanz in gelöster Form auf die zu beschichtende Oberfläche aufgebracht wird und der Film durch Entzug des Lösungsmittels oder Lösemittelgemisches entsteht.

Der Begriff Oberflächenbenetzbarkeit oder Benetzbarkeit wird im Sinne der vorliegenden Anmeldung als Maß für die hydrophilen oder hydrophoben Eigenschaften einer Oberfläche eingesetzt. Durch wässrige Flüssigkeiten gut benetzbare Oberflächen und Substanzen weisen im Allgemeinen eine hohe Hydrophilie auf. Als Maßzahl für die Benetzbarkeit wird der Benetzungswinkel oder Kontaktwinkel angegeben. Hierunter versteht man den Winkel, den eine Tangente an die Tropfenkontur im Drei-Phasen-Punkt zur Oberfläche des Festkörpers bildet und welcher ein Maß für die Benetzbarkeit einer Oberfläche oder Grenzfläche durch eine andere Phase darstellt. Je kleiner der Benetzungswinkel, umso höher die Benetzbarkeit und umso hydrophiler ist die Oberfläche. Eine Benetzbarkeit einer Oberfläche durch Wasser ist insbesondere dann gegeben, wenn der Benetzungswinkel geringer als 90° ist.

Als wässrige Flüssigkeiten oder Lösungen werden im Sinne der vorliegenden Anmeldung alle unter Normalbedingungen flüssigen Substanzen und Substanzgemische angesehen, welche einen Wassergehalt von mehr als 50 Gewichtsprozent besitzen. Dies können Lösungen oder auch homogene oder heterogene Mischungen wie beispielsweise Dispersionen, Emulsionen oder Suspensionen sein. Insbesondere können dies Probenflüssigkeiten, insbesondere Körperflüssigkeiten oder davon abgeleitete Flüssigkeiten wie Blut, Plasma, Serum, Urin, cerebrospinale Flüssigkeit, Tränenflüssigkeit, Dialysat oder ähnliches sein. Die wässrigen Flüssigkeiten können aber auch Salzlösungen, Pufferlösungen, Kalibrationslösungen, Referenzlösungen, Qualitätskontrolllösungen, Spül-oder Reinigungslösungen, Reagenzlösungen oder Lösungen mit standardisierten Analytkonzentrationen, so genannte Standards, sein.
Als organische Lösungsmittel und Lösemittelgemische gelten im Sinne der vorliegenden Anmeldung Lösungsmittel und Lösemittelgemische mit einem Wassergehalt von weniger als 50 Gewichtsprozent.

Durch die erfindungsgemäßen Verfahren und Vorrichtungen kann ein fluidisches System bereitgestellt werden, bei welchem die Benetzungseigenschaften der inneren Oberflächen der einzelnen fluidischen Bauteile aneinander angeglichen werden können. Hierdurch kann das Risiko einer Festsetzung oder Bildung von Gasblasen an den inneren Oberflächen des fluidischen Systems über eine lange Einsatzdauer hinweg reduziert werden. Dies wird erfindungsgemäß dadurch gelöst, dass an den inneren Oberflächen der fluidischen Bauteile beziehungsweise des fluidischen Systems ein dünner Film desselben hydrophilen Polymers vorliegt. Solche dünnen Filme eines hydrophilen Polymers auf den inneren Oberflächen fluidischer Bauteile können durch das erfindungsgemäße Verfahren besonders einfach und kostengünstig hergestellt werden. Weiterhin weisen die nach dem erfindungsgemäßen Verfahren hergestellten Filme des hydrophilen Polymers eine hohe Haftung an den inneren Oberflächen der fluidischen Bauteile und eine hohe Widerstandsfähigkeit gegenüber physikalischen oder chemischen Belastungen auf, so dass sie insbesondere für den Einsatz in diagnostischen Analysegeräten geeignet sind.

Das erfindungsgemäße Verfahren ist durch die Abfolge folgender Schritte gekennzeichnet:
1. Zunächst werden die inneren Oberflächen des fluidischen Bauteils einer physikalisch-chemischen Behandlung unterzogen.
   Hierbei wird zumindest der Teil der inneren Oberflächen des fluidischen Bauteils behandelt, welcher anschließend mit dem Film des hydrophilen Polymers versehen wird. Hierzu ist es nicht unbedingt nötig, dass während dieser Behandlung das Bauteil in der Form vorliegt, in welcher es später als fluidisches Bauteil in einem fluidischen System vorliegt. In bestimmten Ausführungsformen, insbesondere bei flexiblen oder elastischen Bauteilen, kann durch eine Formveränderung des Bauteils vor der Behandlung die Zugänglichkeit der inneren Oberflächen für diese Behandlung erhöht werden. So können beispielsweise Schlauchstücke dadurch einer Vorbehandlung ihrer inneren Oberfläche unterzogen werden, dass zunächst das Schlauchstück umgestülpt wird, so dass die innere Oberfläche nun nach außen weist, anschließend die Behandlung dieser nun nach außen weisenden Oberfläche erfolgt und schließlich das Schlauchstück wiederum umgestülpt wird, so dass die vorbehandelte Oberfläche nun wieder nach innen zeigt. In einer weiteren Ausführungsform kann zunächst die Außenseite eines Schlauchstücks der Behandlung unterzogen werden. Anschließend wird das Schlauchstück umgestülpt, so dass die behandelte Oberfläche des Schlauchstücks zu dessen inneren Oberfläche wird. In weiteren Ausführungsformen ist es möglich, dass zunächst die Oberflächen einzelner Elemente des fluidischen Bauteils der Behandlung unterzogen werden und diese Elemente anschließend zu dem fluidischen Bauteil zusammengefügt werden. Dies kann insbesondere dann vorteilhaft sein, wenn in einem komplexen fluidischen Bauteil die inneren Oberflächen einer solchen Behandlung nicht oder nur schwer zugänglich sind. Durch eine Vorbehandlung der inneren Oberflächen wird insbesondere die Haftung des Films des hydrophilen Polymers auf den inneren Oberflächen und die Widerstandsfähigkeit des aufgebrachten Films gegenüber physikalischen oder chemischen Belastungen verbessert. Unter physikalischen Belastungen kann insbesondere der wiederholte Kontakt über eine längere Zeit mit wässrigen Probenflüssigkeiten, insbesondere mit Körperflüssigkeiten wie beispielsweise Blut, Plasma, Serum oder Harn, verstanden werden. Bei einer solchen physikalischen Belastung über einen längeren Zeitraum kann sich die Schichtstärke des Films des hydrophilen Polymers im Laufe der Zeit reduzieren oder der Film kann sich ganz oder in Teilen von der inneren Oberflächen des fluidischen Bauteils ablösen. Chemische Belastungen können unter anderem ein Kontakt mit aggressiven chemischen Reagenzien wie beispielsweise aggressiven Reinigungslösungen sein. Eine Behandlung der inneren Oberflächen vor dem Aufbringen des Films des hydrophilen Polymers erfolgt hierbei mit physikalisch-chemischen Methoden, vorzugsweise mittels einer Plasmabehandlung. Prinzipiell können jedoch auch andere physikalisch-chemische Methoden zur Vorbehandlung der Membran, beispielsweise eine Ionenstrahlbehandlung oder eine Behandlung mit einer oxidierenden Substanz, beispielsweise einer Natrium-Naphthalin-Lösung, eingesetzt werden. Solche Verfahren zur Oberflächenbehandlung sind bekannt und beispielsweise in "Surface Modification of Poly(tetrafluoroethylene) Film by Chemical Etching, Plasma and Ion Beam Treatments", Kim S., Journal of Applied Polymer Science, 2000, Vol. 77, S. 1913-1920 oder in WO 94/06485 (Chatelier et al.) beschrieben. Als chemische Methoden zu Oberflächenbehandlung kommen vorzugsweise jedoch nicht solche Methoden in Frage, durch welche zusätzliche Zwischenschichten, beispielsweise Haftvermittlerschichten, aufgetragen werden. Als physikalisch-chemische Methoden zu Oberflächenbehandlung gelten in der vorliegenden Anmeldung vielmehr solche Verfahren als bevorzugt, welche zu einer Erhöhung der Reaktivität der Oberflächen führen, ohne in größeren Umfang die chemische Zusammensetzung der Oberflächen zu verändern.
   In einer bevorzugten Ausführungsform erfolgt die Behandlung der inneren Oberflächen des fluidischen Bauteils vor der Herstellung des Filmes des hydrophilen Polymers durch eine Plasmabehandlung. Durch eine Behandlung der inneren Oberflächen mit einem Plasma vor der Aufbringung der Polymerlösung kann sowohl ein ausreichender Haftverbund zwischen den inneren Oberflächen und dem Polymerfilm als auch eine hohe Widerstandsfähigkeit des Polymerfilms gegenüber physikalischen oder chemischen Belastungen erreicht werden. Durch eine solche physikalische Plasmabehandlung kann auf den Einsatz toxischer Chemikalien zur Oberflächenbehandlung verzichtet werden. Eine Vorbehandlung der inneren Oberflächen mit einen Gasplasma führt zu einer homogenen Modifikation der Oberfläche, welche sich nur in geringe Tiefen des Oberflächenmaterials fortsetzt. Bei solchen Plasma-Methoden zur Oberflächenvorbehandlung wird durch eine elektrische Entladung oder durch Einstrahlung elektromagnetischer Felder in eine Gasatmosphäre unter reduziertem Druck ein Gasplasma mit ionisierten Partikeln erzeugt, welches dazu eingesetzt werden kann, auf der Oberflächen reaktive Bereiche zu erzeugen und somit deren Reaktivität zumindest kurzzeitig zu erhöhen, so dass die die Haftung des Polymerfilms an den inneren Oberflächen und dessen Widerstandsfähigkeit gegenüber physikalischen oder chemischen Belastungen erhöht werden kann.
2. In einem nächsten Verfahrensschritt werden die inneren Oberflächen des fluidischen Bauteils mit einer Lösung des hydrophilen Polymers in Kontakt gebracht.
   Dieses In-Kontakt-Bringen der inneren Oberflächen des fluidischen Bauteils mit der Lösung des hydrophilen Polymers kann dabei mit allen dem Fachmann bekannten Methoden wie Durchspülen oder Befüllen des fluidischen Bauteils mit der Lösung, Eintauchen des fluidischen Bauteils in die Lösung oder Aufsprühen der Lösung auf die inneren Oberflächen erfolgen. Hierbei kann die gesamte innere Oberfläche des fluidischen Bauteils mit einem Film des hydrophilen Polymers versehen werden oder auch nur bestimmte Teilbereiche der inneren Oberflächen. Insbesondere können dies Teilbereiche sein, welche später in einem fluidischen System in Kontakt mit Flüssigkeiten stehen.
   In einer bevorzugten Ausführungsform wird in diesem Verfahrensschritt eine Lösung eines hydrophilen Polymer eingesetzt, welches in organischen Lösungsmitteln und Lösemittelgemischen löslich und in wässrigen Lösungen, insbesondere den Probenflüssigkeiten und wässrigen Reinigungs- oder Kalibrierlösungen, weitgehend unlöslich ist. Durch die Verwendung derartiger hydrophiler Polymere ist es insbesondere möglich, eine haltbare und dauerhafte Beschichtung der inneren Oberflächen fluidischer Bauteile effizient ohne zusätzliche chemische Reaktionsschritte vorzunehmen. Durch den Einsatz von in wässrigen Lösungen weitgehend unlöslichen hydrophilen Polymeren kann eine Auflösung des Films in wässrigen Flüssigkeiten weitgehend vermieden werden, so dass derartige Filme eine erhöhte Haltbarkeit in solchen Medien aufweisen und somit insbesondere für den Einsatz in diagnostischen Analysesystemen geeignet sind.
   In einer bevorzugten Ausführungsform wird als hydrophiles Polymer ein Polyether-Polyurethan-Copolymer eingesetzt. Solche Polyether-Polyurethan-Copolymere sind beispielsweise in den US-Patenten US 5,728,762 (Reich et al.) und US 5,932,200 (Reich et al.) beschrieben. Solche bevorzugten Polyether-Polyurethan-Copolymere sind Blockcopolymere mit hydrophilen Bereichen und hydrophoben Bereichen. Durch diese amphiphilen Eigenschaften lösen sich die Polymere einerseits gut in bestimmten organischen Lösungsmitteln und Lösemittelgemischen, organisieren sich anderseits nach Entzug des organischen Lösungsmittels oder Lösemittelgemisches zu in wässrigen Lösungen weitgehend unlöslichen Hydrogelen mit hydrophilen Oberflächeneigenschaften und sind somit für eine erfindungsgemäße Beschichtung besonders geeignet. Solche besonders geeigneten Polyether-Polyurethan-Copolymere können beispielsweise von CardioTech International, Inc., Woburn, MA, U.S.A. bezogen werden.
   Erfindungsgemäß erfolgt die Herstellung des Films dadurch, dass der Film des hydrophilen Polymers direkt ohne weitere Zwischenschichten auf den inneren Oberflächen des fluidischen Bauteils hergestellt wird. Durch die erfindungsgemäße Oberflächenbehandlung im ersten Verfahrensschritt wird diese Oberfläche in einer Weise modifiziert, dass sowohl ein ausreichender Haftverbund zwischen den inneren Oberflächen und dem Polymerfilm als auch eine hohe Widerstandsfähigkeit des Films gegenüber physikalischen oder chemischen Belastungen erreicht werden kann. Hierdurch kann auf eine aufwändige Aufbringung zusätzlicher Zwischenschichten wie beispielsweise Haftvermittlerschichten auf die Oberfläche vor dem Aufbringen des Films des hydrophilen Polymers verzichtet werden.
3. In einem nächsten Verfahrensschritt wird die Lösung des hydrophilen Polymers durch ein gasförmiges Medium derartig ersetzt wird, dass zunächst die inneren Oberflächen des fluidischen Bauteils mit einem Teil der Lösung des hydrophilen Polymers benetzt bleiben. Hierbei wird ein eventuell vorhandener Überschuss der Polymerlösung durch das Einbringen eines Gasvolumens entfernt, so dass eine bestimmte Menge an Polymerlösung auf der zu beschichtenden Oberfläche zurückbleibt. Das Entfernen überschüssiger Polymerlösung kann aktiv oder passiv erfolgen. Insbesondere kann dies dadurch geschehen, dass das gasförmige Medium dadurch eingebracht wird, dass das fluidische Bauteil mit diesem gasförmigen Medium durchspült und somit die überschüssige Polymerlösung durch dieses verdrängt wird. In anderen Ausführungsformen kann dies dadurch erfolgen, dass das fluidische Bauteil aus der Lösung des hydrophilen Polymers entfernt wird und überschüssige Polymerlösung von den zu beschichtenden Oberflächen des fluidischen Bauteils abfließt und durch das umgebende gasförmige Medium, insbesondere Luft, ersetzt wird. Weiterhin ist es durch Einstellung der Menge und/oder Konzentration der auf der Oberfläche zurückbleibenden Polymerlösung möglich, gezielt die Dicke des auf die Oberfläche aufgebrachten Films des hydrophilen Polymers zu definieren und einzustellen.
4. In einem abschließenden Verfahrensschritt wird auf den inneren Oberflächen des fluidischen Bauteils durch Entzug des Lösungsmittels oder Lösemittelgemisches ein Film des hydrophilen Polymers hergestellt.
   In diesem abschließenden Verfahrensschritt wird das Lösungsmittel oder Lösungsmittelgemisch der an den Oberflächen zurückgebliebenen Polymerlösung entzogen, so dass schließlich ein Film des hydrophilen Polymers auf dieser Oberfläche zurückbleibt. Dieser Entzug des Lösungsmittels oder Lösemittelgemisches muss mindestens soweit erfolgen, dass ein mechanisch stabiler Film des hydrophilen Polymers auf der Oberfläche zurückbleibt. Der Entzug des Lösungsmittels oder Lösemittelgemisches kann dabei passiv, beispielsweise durch langsames Verdunsten in die Außenatmosphäre, oder auch aktiv beschleunigt, beispielsweise durch Anlegen eines Vakuums oder Unterdrucks, durch Spülen der Oberfläche mit einem Gas oder durch beschleunigtes Verdunsten durch Temperaturerhöhung, erfolgen.

In bestimmten Ausführungsformen des Verfahrens ist es möglich, dass zunächst die Oberflächen einzelner Bestandteile oder Bereiche des fluidischen Bauteils, insbesondere flüssigkeitsführende Bereiche, mit einem Film des hydrophilen Polymers versehen werden und diese Elemente anschließend zu dem fluidischen Bauteil zusammengefügt werden.

Durch das Vorliegen eines Films eines hydrophilen Polymers auf den inneren Oberflächen werden die physikalischen Oberflächeneigenschaften des fluidischen Bauteils modifiziert. Durch eine solche Beschichtung kann insbesondere erreicht werden, dass die Benetzbarkeit dieser Oberflächen für wässrige Lösungen erhöht und somit das Risiko von Gasblaseneinschlüssen oder -bildungen gesenkt werden kann. Ein weiterer Effekt des Vorliegens eines solchen Polymerfilms auf den inneren Oberflächen fluidischer Bauteile besteht darin, dass durch die Herstellung des Films auf diesen Oberflächen bestehende Unebenheiten dieser Oberflächen im Nano- bis Mikrometerbereich größtenteils ausgeglichen werden können. Dies ist insbesondere beim Einsatz solcher fluidischer Bauteile in diagnostischen Analysesystemen vorteilhaft, da solche Analysesysteme oft ein erhöhtes Kontaminationsrisiko durch aus vorherigen Messungen oder Reinigungen zurückgebliebene Substanzen aufweisen. Solche Substanzen, insbesondere zelluläre oder makromolekulare Substanzen aus Körperflüssigkeiten wie beispielsweise Proteine, lagern bevorzugt an rauen oder schwer zugänglichen Oberflächenbereichen ab. Das erfindungsgemäße Vorliegen eines stabilen Films eines hydrophilen Polymers auf diesen Oberflächen bewirkt, dass das Risiko einer solchen Kontamination über eine lange Einsatzdauer hinweg reduziert werden kann.
In einer bevorzugten Ausführungsform bestehen die inneren Oberflächen des fluidischen Bauteils aus einem Kunststoff. Unter Kunststoffen versteht man im Sinne der vorliegenden Anmeldung und gemäß einer Definition des Normenausschusses "Kunststoffe im DIN" "Materialien, deren wesentliche Bestandteile aus makromolekularen organischen Verbindungen bestehen, die synthetisch oder durch Abwandeln von Naturprodukten entstehen." Sie sind in vielen Fällen unter bestimmten Bedingungen wie Wärme oder Druck schmelz- und formbar. Zu den Kunststoffen gehören ihrer Natur nach auch Kautschuke und Chemiefasern. Kunststoffe eignen sich besonders als Material für fluidische Elemente, da ihre Eigenschaften in vielfacher Weise modifiziert und somit optimal an die zu erfüllenden Aufgaben im fluidischen System angepasst werden können, beispielsweise durch Modifikation ihrer Elastizität. Viele Kunststoffe können in verschiedenen dem Fachmann bekannten Verfahren wie beispielsweise Spritzguss- oder Tiefziehverfahren geformt werden, so dass dadurch die Form fluidischer Bauteile gezielt ihrer Funktion im fluidischen System angepasst werden kann. Folgende Kunststoffe werden unter anderem als Material fluidischer Bauteile eingesetzt: Polyamide, Polycarbonate, Polypropylene, Polyethylene, Polymethylmethacrylate, Polyvinylchloride, Polyetheretherketone, Acrylnitril-Butadien-Copolymere, Styrol-Methmethacryl-Butadien-Copolymere, Naturkautschuke, Silikonkautschuke, Nitrilkautschuke oder Fluorkautschuke.

In einer bevorzugten Ausführungsform beträgt die Dicke des Films des hydrophilen Polymers auf den inneren Oberflächen dieser Bauteile zwischen 0,01 und 50 µm, bevorzugt zwischen 0,01 und 10 µm, besonders bevorzugt zwischen 0,01 und 5 µm. Durch die Aufbringung dünner Polymerfilme auf die inneren Oberfläche fluidischer Bauteile können einerseits die Anforderungen an die Oberflächeneigenschaften der fluidischen Bauteile, insbesondere Benetzbarkeit oder Oberflächenstruktur, erfüllt werden, andererseits ist durch die Herstellung solcher dünnen Filme ein besonders sparsamer und somit wirtschaftlicher Einsatz des hydrophilen Polymers möglich.

Ein weiterer Aspekt der vorliegenden Erfindung betrifft fluidische Systeme zum Transport von Flüssigkeiten, welche aus mehreren fluidischen Bauteilen bestehen, die in einer Weise verbunden sind, dass ein Transport von Flüssigkeiten zwischen diesen fluidischen Bauteilen möglich ist und welche aus zumindest einem sensorischen Element als fluidischem Bauteil und zumindest einem daran angrenzendem weiteren fluidischen Bauteil bestehen, wobei sowohl auf den inneren Oberflächen der flüssigkeitsführenden Bereiche des sensorischen Elements als auch des daran angrenzenden fluidischen Bauteils ein Film desselben hydrophilen Polymers vorliegt. In fluidischen Systemen aus mehreren fluidischen Bauteilen kann es insbesondere an den Kontaktstellen der einzelnen fluidischen Bauteile zu einer Bildung oder Festsetzung von Gasblasen oder einer Ablagerung-kontaminierender Substanzen kommen, an welchen ein sprunghafter Übergang in den Benetzungseigenschaften der inneren Oberflächen der verschiedenen fluidischen Bauteile, beispielsweise auf Grund unterschiedlicher Oberflächenmaterialen, vorhanden ist. Erfindungsgemäß kann dieses Problem dadurch gelöst werden, dass auf den aneinandergrenzenden inneren Oberflächen der einzelnen fluidischen Bauteile ein Film desselben hydrophilen Polymers, vorliegt. Durch das Vorliegen eines Films desselben hydrophilen Polymers auf den inneren Oberflächen der einzelnen fluidischen Bauteile werden so die physikalischen Eigenschaften dieser Bauteile, insbesondere deren Benetzungseigenschaften, aneinander angeglichen und somit Kontaminationen weitgehend vermieden.
Erfindungsgemäß muss nicht auf den gesamten inneren Oberflächen des fluidischen Systems ein Film eines hydrophilen Polymers vorliegen. In vielen Fällen ist es ausreichend, wenn ein solcher Film nur in bestimmten Bereichen des fluidischen Systems beziehungsweise auf den inneren Oberflächen oder Teilen dieser Oberflächen von bestimmten fluidischen Bauteilen vorliegt. Werden fluidische Systeme in Analysesystemen, insbesondere in diagnostischen Analysesystemen, eingesetzt, ist es zur exakten und fehlerfreien Bestimmung der Messwerte oft ausreichend, die Bereiche des fluidischen Systems mit einem Film eines hydrophilen Polymers zu versehen, welche sich, ausgehend von der Probenaufgabevorrichtung, auf dem Transportweg vor dem jeweiligen sensorischen Element oder im flüssigkeitsführenden Bereich des sensorischen Elements selbst befinden. Weitere bevorzugt mit einem Film eines hydrophilen Polymers zu versehende Bereiche des fluidischen Systems sind die Bereiche, welche sich im fluidischen System ebenfalls vor den sensorischen Elementen befinden, aber keine Probenflüssigkeit, sondern beispielsweise Kalibrier- oder Reinigungsmedien transportieren und oft vor den sensorischen Elementen in den Transportweg der Probenflüssigkeit einmünden. Die den sensorischen Elementen nachgeschalteten Bereiche des fluidischen Systems, beispielsweise die Transportleitungen zu den Abfallbehältern, müssen nicht notwendigerweise einen Film des hydrophilen Polymers aufweisen, da Gasblasen oder Kontaminationen in diesen Bereichen keine negativen Auswirkungen auf eine Messwertbestimmungen haben.

Ein weiterer Aspekt der vorliegenden Erfindung betrifft Verfahren zur Herstellung solcher fluidischer Systeme, welche zumindest in Teilen auf ihren inneren Oberflächen einen Film eines hydrophilen Polymers aufweisen.
In einer bevorzugten Ausführungsform erfolgt die Herstellung eines solchen fluidischen Systems dadurch, dass die Herstellung des Filmes des hydrophilen Polymers zunächst auf den inneren Oberflächen einzelner fluidischer Bauteile oder in kleinerer Baugruppen solcher fluidischer Bauteile erfolgt, welche anschließend zu dem fluidischen System zusammengefügt werden. Dieses Verfahren kann insbesondere in Fällen eingesetzt werden, in welchen die Beschichtung des gesamten fluidischen Systems oder großer Teile davon wirtschaftlich nicht sinnvoll oder technisch nicht oder nur schwer möglich ist, beispielsweise dadurch, dass einzelne Teilbereiche des Systems für eine solche Beschichtung nicht oder nur sehr schwer zugänglich sind. Dies können beispielsweise Teilbereiche des fluidischen Systems hinter Ventilen oder sehr enge Flüssigkeitstransportwege sein. Um dennoch einen Film eines hydrophilen Polymers in solchen Bereichen des fluidischen Systems herstellen zu können, werden die einzelnen fluidischen Bauteile, deren innere Oberflächen mit einem Film eines hydrophilen Polymers versehen werden sollen, zunächst einzeln mit einem solchem Film versehen und anschließend zu einem fluidischen System zusammengefügt. Dadurch können auch Teile des Systems, welche im zusammengefügten System einer solchen Beschichtung nicht oder nur schwer zugänglich sind, auf einfache Weise mit einem Film eines hydrophilen Polymers versehen werden.
In einer weiteren bevorzugten Ausführungsform erfolgt die Herstellung eines solchen fluidischen Systems dadurch, dass zunächst die inneren Oberflächen einzelner fluidischer Bauteile physikalisch-chemisch vorbehandelt werden, diese Bauteile anschließend zu einem fluidischen System zusammengefügt werden und die weiteren erfindungsgemäßen Verfahrensschritte an diesem zusammengefügtem fluidischen System erfolgen. Dieses Verfahren kann insbesondere in den Fällen eingesetzt werden, in welchen eine physikalisch-chemische Vorbehandlung des gesamten fluidischen Systems oder großer Teile davon wirtschaftlich nicht sinnvoll oder technisch nicht oder nur schwer möglich ist, beispielsweise dadurch, dass einzelne Teilbereiche des Systems für eine solche Vorbehandlung nicht oder nur sehr schwer zugänglich sind. Bei manchen Vorbehandlungsverfahren, insbesondere bei einer Plasmabehandlung, tritt in manchen Fällen das Problem auf, dass die Wirkung der Vorbehandlung sich nur auf leicht zugängliche Bereiche des fluidischen Systems beschränkt, während schwer zugängliche Bereiche des fluidischen Systems nicht oder nur kaum dieser Vorbehandlung unterzogen werden. Um dieses Problem zu überwinden, wird hiermit ein Verfahren bereitgestellt, welches dieses Problem dadurch löst, dass nicht das ganze fluidische System oder große Teile dessen einer solchen Vorbehandlung unterzogen wird, sondern die Vorbehandlung zunächst nur auf einzelne fluidische Bauteile oder Teile derer angewendet wird, welche dadurch leichter vorbehandelt werden können. So können beispielsweise die einzelne Elemente eines Ventils oder Segmente eines langen Schlauchstücks einzeln vorbehandelt werden und anschließend zusammengefügt werden. Die weiteren Schritte zur Herstellung des Films des hydrophilen Polymers auf den inneren Oberflächen des fluidischen Systems sind oft nicht mehr kritisch und können im zusammengefügten System durchgeführt werden.
In den vorgenannten Ausführungsformen kann die Herstellung des Films auch dadurch erfolgen, dass die für einzelne fluidische Bauteile beschriebenen Verfahrensschritte an kleineren Baugruppen mehrerer solcher fluidischen Bauteile durchgeführt werden.

Beispielsweise können fluidische Bauteile wie ein Schlauch und ein Winkelstück als kleine Baugruppe vor Beginn des erfindungsgemäßen Verfahrens zusammengefügt werden und durchlaufen die nachfolgenden Schritte zur Herstellung eines Films eines hydrophilen Polymers auf den inneren Oberflächen als gemeinsame kleine Baugruppe.

Ein weiterer Aspekt der vorliegenden Erfindung betrifft Analysesysteme zur Bestimmung mindestens eines physikalischen oder chemischen Parameters einer Flüssigkeit, welche mindestens ein sensorisches Element zur Bestimmung mindestens eines physikalischen oder chemischen Parameters der Flüssigkeit und ein erfindungsgemäßes fluidisches System zum Transport von Flüssigkeiten zum sensorischen Element hin und/oder vom sensorischen Element weg enthalten.
In einem solchen Analysesystem können alle zuvor beschriebenen Ausführungsformen erfindungsgemäßer fluidischer Systeme eingesetzt werden. Solche Analysesysteme können prinzipiell zur Analyse aller Arten von Flüssigkeiten eingesetzt, besonders bevorzugt zur Analyse wässriger Flüssigkeiten. In einer bevorzugten Ausführungsform werden solche Analysesysteme zur Analyse von physiologischen Flüssigkeiten wie Blut, Plasma, Serum, Harn, cerebrospinale Flüssigkeit, Tränenflüssigkeit oder flüssigen biologischen Proben wie Zellensuspensionen, Zellüberständen, Zellextrakten, Gewebeaufschlüssen oder ähnlichen eingesetzt. Besonders bevorzugt werden sie zur Analyse von Blut, Serum, Plasma oder Harn eingesetzt.
In einer bevorzugten Ausführungsform liegt auch auf den inneren Oberflächen der flüssigkeitsführenden Bereiche eines oder mehrerer sensorischer Elemente des Analysesystems ein Film des hydrophilen Polymers vor. Diese flüssigkeitskeitsführenden Bereiche sensorischer Elemente stehen direkt mit der zu analysierenden Flüssigkeit in Kontakt. Das Vorliegen eines Films eines hydrophilen Polymers in diesen Bereichen ist besonders vorteilhaft, da hiermit das Risiko einer möglichen Messwertverfälschung durch das Vorhandensein von Gasblasen oder kontaminierenden Substanzen im sensorischen Element verringert werden kann. In besonders bevorzugten Ausführungsformen kann durch das Vorliegen eines Films desselben hydrophilen Polymers sowohl auf den flüssigkeitsführenden Bereichen des sensorischen Elements als auch den daran angrenzenden Teilen des restlichen fluidischen Systems ein solches Risiko weiter reduziert werden.
In einer weiteren bevorzugten Ausführungsform enthält das Analysesystem mehrere sensorische Elemente zur Bestimmung verschiedener physikalischer und/oder chemischer Parameter einer Flüssigkeit, welche meist für viele Bestimmungen eingesetzt werden. Neben mehr- und vielfach einsetzbaren sensorischen Elementen können jedoch auch nur einmal einsetzbare sensorische Elemente benutzt werden. Die verschiedenen Elemente sind hierbei meist durch fluidische Bauteile miteinander verbunden, können jedoch auch direkt miteinander in Kontakt stehen. Verschiedene sensorische Elemente können hierbei auch zu sensorischen Modulen zusammengefasst werden, welche ein gemeinsames fluidisches Teilsystem besitzen.

### Beispiel 1: Herstellung eines Films eines hydrophilen Polymers auf einem plasmavorbehandelten fluidischen Element am Beispiel eines Gehäusekanals

Als Beispiel eines fluidisches Bauteils wird der Gehäusekanal eines sensorischen Elements, beispielsweise einer Sauerstoffelektrode, im folgenden Beispiel beschrieben. Solche Gehäusekanäle entsprechen im Wesentlichen den flüssigkeitsführenden Bereichen sensorischer Elemente, welche mit Flüssigkeiten direkt in Kontakt stehen und beinhalten insbesondere den flüssigkeitszuführenden und -abführenden Probenkanal innerhalb des sensorischen Elements.

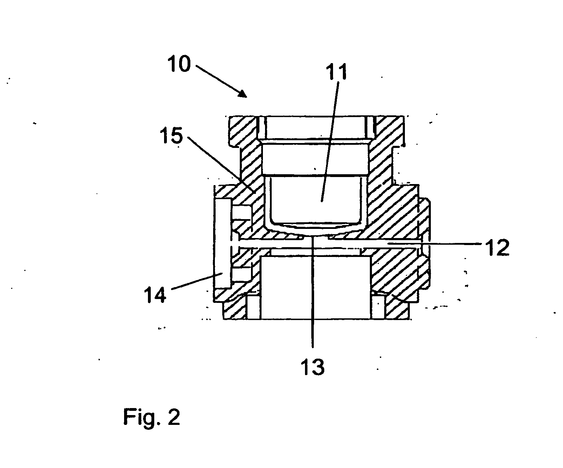

Figur 2 zeigt exemplarisch einen Schnitt durch den sensorischen Bereich eines solchen Sensorelements am Beispiel eines miniaturisierten Sauerstoff-Sensors vom Clark-Typ, wie er beispielsweise in den OMNI-Analysesystemen von Roche Diagnostics eingesetzt wird. Diese sensorischen Elemente (10) umfassen neben dem eigentlichen Sensor (11) mit Innenelektrolytraum und den darin befindlichen Elektroden einen Proben- oder Gehäusekanal (12) zum Transport und zur Bereitstellung der Probe. Zwischen Innenelektrolytraum und Probenkanal befindet sich eine gasdurchlässige und weitgehend ionen- und flüssigkeitsundurchlässige Kunststoff-Membran (13), welche Innenelektrolytraum und Probenkanal trennt. Das Gehäuse des sensorischen Elements (15) und damit auch die Wandungen des Gehäusekanals (12) bestehen im dargestellten Fall aus einem transparenten Kunststoff, beispielsweise einem Methylmethacrylat-Butadien-StyrolCopolymer.
Zur Vorbehandlung des Gehäusekanals vor der eigentlichen Hydrogelbeschichtung wird das Gehäuse (15) auf einer Trägerplatte fixiert und in eine Plasmaanlage von Typ V15-G der Firma Plasmafinish eingebracht und analog den Herstellerangaben für wenige Minuten bei 2,45 GHz mit einem Heliumplasma behandelt. Das Plasma kann im dargestellten Fall sowohl durch die beidseitigen Öffnungen des Gehäusekanals (12) als auch durch die Öffnung im Gehäuse, welche später durch die Membran (13) abgedeckt wird, an die Innenseiten des Gehäusekanals gelangen. Innerhalb von ca. 5 - 60 Minuten nach Ende der Plasmabehandlung wird das plasmabehandelte Gehäuse von der Trägerplatte abgenommen und in einer Gehäuseaufnahme fixiert. In dieser Gehäuseaufnahme werden nun zu- und abführende Flüssigkeitsleitungen an die beiden Enden des Gehäusekanals (12) gebracht. Die Flüssigkeitsleitungen werden mittels Pressverbindungen über die am Gehäuse (15) vorhandenen quetschbaren Dichtung (14) oder eine an der Flüssigkeitsleitung vorhandene Dichtung abgedichtet. Durch die Flüssigkeitsleitungen und somit auch durch den Gehäusekanal (12) wird eine Lösung des hydrophilen Polymers X1 für einige Minuten gesaugt. X1 bezeichnet ein hydrophiles Polymer vom Polyether-Polyurethan-Copolymer-Typ, welches beispielsweise von Cardiotech International, 78 E Olympia Avenue, Wobum MA 01801-2057, USA als "Hydrophilic Polyether Polyurethane" bezogen werden kann und eine Wasseraufnahmerate von 50 % und eine Expansionsrate durch Quellung von 60 % aufweist. Zur Herstellung einer Lösung des hydrophilen Polymers X1 wird eine definierte Menge des Hydrogelgranulats X1 in einer Ethanol-Wassermischung unter Rühren gelöst. Anschließend wird die Lösung des hydrophilen Polymers X1 aus den Flüssigkeitsleitungen und dem Gehäusekanal (12) entfernt und für einige Minuten Luft durch die den Flüssigkeitsleitungen und den Gehäusekanal gesaugt. Hierdurch erfolgt eine Trocknung der Hydrogelschicht auf den Innenwänden des Gehäusekanals (12) so dass als Ergebnis des Beschichtungsverfahrens erfindungsgemäß ein Film des hydrophilen Polymers auf den Innenwänden des Gehäusekanals (12) vorliegt.

## Patentansprüche

1. Fluidisches System bestehend aus zumindest einem sensorischen Element als fluidisches Bauteil und zumindest einem daran angrenzendem weiteren fluidischen Bauteil,
**dadurch gekennzeichnet, dass**
sowohl auf den inneren Oberflächen der flüssigkeitsführenden Bereiche des sensorischen Elements als auch des daran angrenzenden fluidischen Bauteils ein Film desselben hydrophilen Polymers vorliegt.

2. Fluidisches System gemäß Anspruch 1,
**dadurch gekennzeichnet, dass**
das fluidische Bauteil ein Bauteil aus der Gruppe umfassend die Elemente Kanal, Schlauch, Rohr, Ventil, Verteilerstück, Ankopplungselement, Flüssigkeitsaufnahmevorichtung, Nippel, flüssigkeitsführender Bereich eines sensorischen Elements oder Flüssigkeitsbehälter ist.

3. Fluidisches System gemäß Anspruch 1 oder 2,
**dadurch gekennzeichnet, dass**
die Dicke des Films des hydrophilen Polymers zwischen 0,01 und 50 µm, bevorzugt zwischen 0,01 und 10 µm, besonders bevorzugt zwischen 0,01 und 5 µm beträgt.

4. Fluidisches System gemäß einem der Ansprüche 1 bis 3,
**dadurch gekennzeichnet, dass**
das hydrophile Polymer in organischen Lösungsmitteln oder Lösemittelgemischen löslich und in wässrigen Lösungen weitgehend unlöslich ist.

5. Fluidisches System gemäß einem der Ansprüche 1 bis 4,
**dadurch gekennzeichnet, dass**
das hydrophile Polymer ein Blockcopolymer mit hydrophilen Bereichen und hydrophoben Bereichen ist.

6. Fluidisches System gemäß einem der Ansprüche 1 bis 5,
**dadurch gekennzeichnet, dass**
das hydrophile Polymer ein Polyether-Polyurethan-Copolymer ist.

7. Sensorisches Modul zur Bestimmung physikalischer und/oder chemischer Parameters einer Flüssigkeit, umfassend mehrere sensorische Elemente sowie ein fluidisches Teilsystem zum Transport der Flüssigkeiten zu den sensorischen Elementen hin bzw. von diesen weg,
**dadurch gekennzeichnet, dass**
zumindest ein fluidisches System gemäß einem der Ansprüche 1 - 6 enthalten ist.

8. Sensorisches Modul gemäß Anspruch 7,
**dadurch gekennzeichnet, dass**
es als Kassette oder sensorisches Array ausgebildet ist, womit eine Mehrzahl von physikalischen oder chemischen Parametern weitgehend gleichzeitig anband einer einzigen Probe ermittelt werden kann.

9. Sensorisches Modul gemäß Anspruch 7 oder 8,
**dadurch gekennzeichnet, dass**
es ein fluidisches System mit einem fluidischen Eingang, einem durchgängigen oder auch verzweigten fluidischen Transportraum aus einem oder mehreren Kanälen und einen fluidischen Ausgang aufweist, wobei insbesondere mehrere sensorische Elemente in Kontakt mit dem gemeinsamen fluidischen Transportraum stehen, welcher somit die Funktion eines gemeinsamen Probenkanals erfüllt.

10. Analysesystem zur Bestimmung mindestens eines physikalischen oder chemischen Parameters einer Flüssigkeit, umfassend mindestens ein sensorisches Element zur Bestimmung mindestens eines physikalischen oder chemischen Parameters der Flüssigkeit und ein fluidisches System zum Transport von Flüssigkeiten zum sensorischen Element hin und/oder vom sensorischen Element weg,
**dadurch gekennzeichnet, dass**
zumindest ein fluidisches System gemäß einem der Ansprüche 1 - 6 enthalten ist.

11. Analysesystem gemäß Anspruch 10,
**dadurch gekennzeichnet, dass**
die zu analysierende Flüssigkeit eine physiologische Flüssigkeit, insbesondere Blut, Serum, Plasma oder Harn, ist.

12. Verfahren zur Herstellung eines fluidisches Systems, bestehend aus zumindest einem sensorischen Element als fluidisches Bauteil und zumindest einem daran angrenzendem weiteren fluidischen Bauteil gemäß einem der Ansprüche 1- 6,
**dadurch gekennzeichnet, dass**
die Herstellung eines Films desselben hydrophilen Polymers dadurch erfolgt, dass
a) die inneren Oberflächen der fluidischen Bauteile einer physikalisch-chemischen Vorbehandlung unterzogen werden,
b) die inneren Oberflächen der fluidischen Bauteile mit einer Lösung desselben hydrophilen Polymers in Kontakt gebracht werden,
c) die Lösung des hydrophilen Polymers durch ein gasförmiges Medium derartig ersetzt wird, dass zunächst die inneren Oberflächen der fluidischen Bauteile mit einem Teil der Lösung des hydrophilen Polymers benetzt bleiben und
d) ein Film des hydrophilen Polymers durch Entzug des Lösungsmittels auf den inneren Oberflächen der fluidischen Bauteile verbleibt und
die Herstellung des Filmes des hydrophilen Polymers zunächst auf den inneren Oberflächen einzelner fluidischer Bauteile oder kleinerer Baugruppen solcher fluidischer Bauteile erfolgt, welche anschließend zu dem fluidischen System gemäß einem der Ansprüche 1-6 zusammengefügt werden.

13. Verfahren zur Herstellung eines fluidisches Systems, bestehend aus zumindest einem sensorischen Element als fluidisches Bauteil und zumindest einem daran angrenzendem weiteren fluidischen Bauteil gemäß einem der Ansprüche 1 - 6,
**dadurch gekennzeichnet, dass**
die Herstellung eines Films desselben hydrophilen Polymers dadurch erfolgt, dass
a) die inneren Oberflächen der fluidischen Bauteile einer physikalisch-chemischen Vorbehandlung unterzogen werden,
b) die inneren Oberflächen der fluidischen Bauteile mit einer Lösung desselben hydrophilen Polymers in Kontakt gebracht werden,
c) die Lösung des hydrophilen Polymers durch ein gasförmiges Medium derartig ersetzt wird, dass zunächst die inneren Oberflächen der fluidischen Bauteile mit einem Teil der Lösung des hydrophilen Polymers benetzt bleiben und
d) ein Film des hydrophilen Polymers durch Entzug des Lösungsmittels auf den inneren Oberflächen der fluidischen Bauteile verbleibt und
die Vorbehandlung gemäß Verfahrensschritt a) zunächst auf den inneren Oberflächen einzelner fluidischer Bauteile oder kleinerer Baugruppen solcher fluidischer Bauteile erfolgt, diese fluidischen Bauteile oder Baugruppen anschließend zu dem fluidischen System zusammengefügt werden und die Verfahrensschritte b) bis d) an diesem zusammengefügtem fluidischen System erfolgen.

14. Verfahren gemäß Anspruch 12 oder 13,
**dadurch gekennzeichnet, dass**
die physikalisch-chemische Vorbehandlung eine Plasmabehandlung ist.

15. Verfahren gemäß einem der Ansprüche 12 bis 14,
**dadurch gekennzeichnet, dass**
der Film des hydrophilen Polymers direkt ohne weitere Zwischenschichten auf den inneren Oberflächen des fluidischen Bauteils hergestellt wird.

## Claims

1. Fluidic system consisting of at least one sensory element as a fluidic component and at least one additional adjoining fluidic component,
**characterized in that**
a film of the same hydrophilic polymer is present on the inner surfaces of the liquid-conveying areas of the sensory element as well as of the adjoining fluidic component.

2. Fluidic system according to claim 1,
**characterized in that**
the fluidic component is a component from the group comprising the elements channel, tube, pipe, valve, distributor, coupling element, liquid receiving device, nipple, liquid-conveying area of a sensory element or liquid container.

3. Fluidic system according to claim 1 or 2,
**characterized in that**
the thickness of the film of hydrophilic polymer is between 0.01 and 50 µm, preferably between 0.01 and 10 µm, particularly preferably between 0.01 and 5 µm.

4. Fluidic system according to one of the claims 1 to 3,
**characterized in that**
the hydrophilic polymer is soluble in organic solvents or solvent mixtures and substantially insoluble in aqueous solutions.

5. Fluidic system according to one of the claims 1 to 4,
**characterized in that**
the hydrophilic polymer is a block copolymer with hydrophilic regions and hydrophobic regions.

6. Fluidic system according to one of the claims 1 to 5,
**characterized in that**
the hydrophilic polymer is a polyether-polyurethane copolymer.

7. Sensory module for determining physical and/or chemical parameters of a liquid comprising several sensory elements as well as a fluidic subsystem for transporting the liquids to and away from the sensory element,
**characterized in that**
it contains at least one fluidic system according to one of the claims 1 - 6.

8. Sensory module according to claim 7,
**characterized in that**
it is designed as a cassette or sensory array with which a plurality of physical or chemical parameters can be determined substantially simultaneously with a single sample.

9. Sensory module according to claim 7 or 8,
**characterized in that**
it has a fluidic system with a fluidic inlet, a continuous or branched fluidic transport space comprising one or more channels and a fluidic outlet, wherein in particular several sensory elements are in contact with the common fluidic transport space which thus fulfils the function of a common sample channel.

10. Analytical system for determining at least one physical or chemical parameter of a liquid comprising at least one sensory element for determining at least one physical or chemical parameter of the liquid and a fluidic system for transporting liquids to the sensory element and/or away from the sensory element,
**characterized in that**
it contains at least one fluidic system according to one of the claims 1 - 6.

11. Analytical system according to claim 10,
**characterized in that**
the liquid to be analysed is a physiological liquid, in particular blood, serum, plasma or urine.

12. Process for producing a fluidic system consisting of at least one sensory element as a fluidic component and at least one additional adjoining fluidic component according to one of the claims 1 - 6,
**characterized in that**
a film of the same hydrophilic polymer is produced by
a) subjecting the inner surfaces of the fluidic components to a physico-chemical pretreatment,
b) bringing the inner surfaces of the fluidic components into contact with a solution of the same hydrophilic polymer,
c) replacing the solution of the hydrophilic polymer by a gaseous medium in such a manner that firstly the inner surfaces of the fluidic components remain wetted with a part of the solution of the hydrophilic polymer and
d) a film of the hydrophilic polymer remains on the inner surfaces of the fluidic components by removing the solvent and
the film of the hydrophilic polymer is firstly produced on the inner surfaces of individual fluidic components or smaller subassemblies of such fluidic components which are subsequently assembled to form the fluidic system according to one of the claims 1 - 6.

13. Process for producing a fluidic system consisting of at least one sensory element as a fluidic component and at least one additional adjoining fluidic component according to one of the claims 1 - 6,
**characterized in that**
a film of the same hydrophilic polymer is produced by
a) subjecting the inner surfaces of the fluidic components to a physico-chemical pretreatment,
b) bringing the inner surfaces of the fluidic components into contact with a solution of the same hydrophilic polymer,
c) replacing the solution of the hydrophilic polymer by a gaseous medium in such a manner that firstly the inner surfaces of the fluidic components remain wetted with a part of the solution of the hydrophilic polymer and
d) a film of the hydrophilic polymer remains on the inner surfaces of the fluidic components by removing the solvent and the pretreatment according to process step (a) is firstly carried out on the inner surfaces of individual fluidic components or smaller subassemblies of such fluidic components, these fluidic components or subassemblies are subsequently assembled to form the fluidic system and the process steps b) to
d) being carried out on this assembled fluidic system.

14. Process according to claim 12 or 13,
**characterized in that**
the physico-chemical pretreatment is a plasma treatment.

15. Process according to one of the claims 12 to 14,
**characterized in that**
the film of the hydrophilic polymer is produced directly without additional intermediate layers on the inner surfaces of the fluidic component.

## Revendications

1. Système fluidique comportant au moins un élément sensoriel en tant que composant fluidique et au moins un autre composant fluidique adjacent à celui-ci,
**caractérisé en ce que**
un film d'un même polymère hydrophile est présent à la fois sur les surfaces internes des zones parcourues par le liquide de l'élément sensoriel et du composant fluidique adjacent à celui-ci.

2. Système fluidique selon la revendication 1,
**caractérisé en ce que**
le composant fluidique est un composant du groupe comprenant les éléments canal, tuyau, tube, vanne, manifold, élément de couplage, raccord, dispositif d'admission de liquide, zone parcourue par le liquide d'un élément sensoriel ou réservoir de liquide.

3. Système fluidique selon la revendication 1 ou 2,
**caractérisé en ce que**
l'épaisseur du film de polymère hydrophile est comprise entre 0,01 et 50 µm, de préférence entre 0,01 et 10 µm, plus particulièrement entre 0,01 et 5 µm.

4. Système fluidique selon l'une des revendications 1 à 3,
**caractérisé en ce que**
le polymère hydrophile est soluble dans les solvants ou mélanges de solvants organiques et sensiblement insoluble dans les solutions aqueuses.

5. Système fluidique selon l'une des revendications 1 à 4,
**caractérisé en ce que**
le polymère hydrophile est un copolymère séquencé comportant des zones hydrophiles et des zones hydrophobes.

6. Système fluidique selon l'une des revendications 1 à 5,
**caractérisé en ce que**
le polymère hydrophile est un copolymère polyéther-polyuréthane.

7. Module sensoriel pour la détermination de paramètres physiques et/ou chimiques d'un liquide, comprenant plusieurs éléments sensoriels ainsi qu'un sous-système fluidique pour le transport des liquides vers lesdits éléments sensoriels, respectivement en direction opposée,
**caractérisé en ce que**
au moins un système fluidique selon l'une des revendications 1 à 6 est inclus.

8. Module sensoriel selon la revendication 7,
**caractérisé en ce que**
il est conçu sous forme de cassette ou de matrice sensorielle, ce qui permet de déterminer de manière sensiblement simultanée une majorité de paramètres physiques ou chimiques à l'aide d'un seul échantillon.

9. Module sensoriel selon la revendication 7 ou 8,
**caractérisé en ce que**
il comporte un système fluidique avec une entrée fluidique, un espace de transport fluidique traversant ou bien ramifié constitué d'un ou de plusieurs canaux et une sortie fluidique, où notamment plusieurs éléments sensoriels sont en contact avec ledit espace de transport fluidique commun, lequel remplit ainsi la fonction d'un canal d'échantillon commun.

10. Système d'analyse pour la détermination d'au moins un paramètre physique ou chimique d'un liquide, comprenant au moins un élément sensoriel pour la détermination d'au moins un paramètre physique ou chimique d'un liquide et un système fluidique pour le transport de liquides vers ledit élément sensoriel et/ou en direction opposée,
**caractérisé en ce que**
au moins un système fluidique selon l'une des revendications 1 à 6 est inclus.

11. Système d'analyse selon la revendication 10,
**caractérisé en ce que**
le liquide à analyser est un liquide physiologique, en particulier du sang, du sérum, du plasma ou de l'urine.

12. Procédé de fabrication d'un système fluidique comportant au moins un élément sensoriel en tant que composant fluidique et au moins un autre composant fluidique adjacent à celui-ci selon l'une des revendications 1 à 6,
**caractérisé en ce que**
la réalisation d'un film d'un même polymère hydrophile s'effectue
a) en soumettant les surfaces internes des composants fluidiques à un prétraitement physico-chimique,
b) en mettant en contact les surfaces internes des composants fluidiques avec une solution d'un même polymère hydrophile,
c) en remplaçant la solution de polymère hydrophile par un milieu gazeux de telle sorte que ce soient d'abord les surfaces internes des composants fluidiques qui restent humectées par une partie de la solution de polymère hydrophile et
d) un film de polymère hydrophile demeure sur les surfaces internes des composants fluidiques par élimination du solvant,
et
la réalisation du film de polymère hydrophile s'effectue d'abord sur les surfaces internes de différents composants fluidiques ou d'ensembles plus petits de tels composants fluidiques, lesquels sont ensuite assemblés pour former le système fluidique selon l'une des revendications 1 à 6.

13. Procédé de fabrication d'un système fluidique comportant au moins un élément sensoriel en tant que composant fluidique et au moins un autre composant fluidique adjacent à celui-ci selon l'une des revendications 1 à 6,
**caractérisé en ce que**
la réalisation d'un film d'un même polymère hydrophile s'effectue
a) en soumettant les surfaces internes des composants fluidiques à un prétraitement physico-chimique,
b) en mettant en contact les surfaces internes des composants fluidiques avec une solution d'un même polymère hydrophile,
c) en remplaçant la solution de polymère hydrophile par un milieu gazeux de telle sorte que ce soient d'abord les surfaces internes des composants fluidiques qui restent humectées par une partie de la solution de polymère hydrophile et
d) un film de polymère hydrophile demeure sur les surfaces internes des composants fluidiques par élimination du solvant,
et
le prétraitement selon l'étape a) s'effectue d'abord sur les surfaces internes de différents composants fluidiques ou d'ensembles plus petits de tels composants fluidiques, ces composants ou ensembles fluidiques sont ensuite assemblés pour former le système fluidique et les étapes b) à d) s'effectuent sur ce système fluidique assemblé.

14. Procédé selon la revendication 12 ou 13,
**caractérisé en ce que**
le prétraitement physico-chimique est un traitement au plasma.

15. Procédé selon l'une des revendications 12 à 14,
**caractérisé en ce que**
le film de polymère hydrophile est réalisé directement, sans d'autres couches intermédiaires, sur les surfaces internes du composant fluidique.
